Europäisches Patentamt

European Patent Office

Office européen des brevets

(11) Veröffentlichungsnummer: **0 256 365**
**A1**

# (12) EUROPÄISCHE PATENTANMELDUNG

(21) Anmeldenummer: 87110842.9

(22) Anmeldetag: 27.07.87

(51) Int. Cl.⁴: **A61K 9/08** , A61K 47/00 , A61K 31/415

(30) Priorität: 08.08.86 DE 3626908

(43) Veröffentlichungstag der Anmeldung:
24.02.88 Patentblatt 88/08

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Serno, Peter, Dr.**
**An der Ruthen 3**
**D-5000 Köln 80(DE)**
Erfinder: **Detzer, Klaus, Dr.**
**Überdorf 6**
**D-5307 Wachtberg-Grimmersdorf(DE)**
Erfinder: **Winter, Manfred, Dr.**
**Roggendorfstrasse 49**
**D-5000 Köln 80(DE)**

(54) **Parenterale Lösung.**

(57) Parenterale Lösungen von 5-Amino-2-[1-(3,4-Dichlorophenyl)-ethyl]-2,4-dihydro-3H-pyrazol-3-on enthalten modifizierte Gelatine.

EP 0 256 365 A1

## Parenterale Lösung

Die parenterale Applikation von Arzneistoffen, im wesentlichen die intravenöse Applikation, ist nur in gelöster Form möglich. Daher bereitet die Formulierung von Injektions-und Infusionslösungen bei Arzneistoffen geringer Wasserlöslichkeit regelmäßig Schwierigkeiten.

Im Falle unzureichender Löslichkeit in Wasser wurden bislang bei der Formulierung parenteraler Lösungen schwer löslicher Arzneistoffe organische Lösungsmittel wie Propylenglykol, Polyethylenglykol, Ethanol, Glycerin-Polyethylenglykolricinoleat (Cremophor® EL) oder Polyoxyethylensorbitanfettsäureester (Tween®) zur Erhöhung der Löslichkeit zugesetzt. Die Effektivität dieser Maßnahme wird allerdings dadurch begrenzt, daß Lösungsmittel nur in niederen Konzentrationen angewendet werden können, da höhere Konzentration zu unerwünschten Nebenwirkungen wie Injektionsschmerz, Thrombophlebitis und Venenverödung führen. Darüber hinaus führen einige Lösungsmittel zu Nebenwirkungen wie anaphylaktische Schocks und Hämolyse.

Eine andere Möglichkeit der Lösungsvermittlung von Arzneistoffen geringer Wasserlöslichkeit besteht darin, den Arzneistoff in der Fettphase in einer Emulsion zu lösen (US 4 073 943). Dieses Verfahren setzt allerdings eine sehr gute Löslichkeit des Arzneistoffs in physiologisch verträglichen Ölen wie Sojabohnenöl voraus, die nur in den seltensten Fällen gewährleistet ist.

Die in US 4 158 707 beschriebene Lösungsvermittlung mit einer Kombination aus Gallensäure und Lipoid hat den Nachteil einer begrenzten Haltbarkeit der Lösungsvermittler, insbesondere bei höheren Temperaturen sowie von Nebenwirkungen wie Erbrechen, Hämolyse und Cholestasis bei Gaben in höheren Dosen.

Der bekannte Wirkstoff 5-Amino-2-[1-(3,4-dichlorophenyl)-ethyl]-2,4-di-hydro-3H-pyrazol-3-on (nach INN: Muzolimin) (DE-OS 23 19 280) wird in parenteralen Zubereitungen aus den genannten Gründen nicht appliziert.

Es wurden parenterale Lösungen von 5-Amino-2-[1-(3,4-dichlorophenyl)-ethyl]-2,4-di-hydro-3H-pyrazol-3-on gefunden, die modifizierte Gelatine enthalten.

Die erfindungsgemäßen parenteralen Lösungen können Muzolimin in Konzentrationen von 0,1 g bis 100 g, bevorzugt 1 g bis 10 g, pro Liter enthalten.

Parenterale Lösungen sind hierbei im wesentlichen intravenöse Applikationsformen von Muzolimin, im besonderen Injektions-und Infusionslösungen. Für die erfindungsgemäßen parenteralen Lösungen verwendet man im allgemeinen modifizierte Gelatine mit einem Molekulargewicht im Bereich von 10.000 und 50.000 wie Oxypolygelatine oder Polymerisat abgebauter succinoylierter Gelatine.

Die modifizierte Gelatine ist an sich bekannt und kann beispielsweise Oxypolygelatine, Polygeline oder Succinoyl-Gelatine darstellen.

Oxypolygelatine wird durch Decalcifizieren von Gelatine, Kondensation mit Glyoxal und Oxidation mit Wasserstoffperoxid hergestellt und ist von Campbell, D. H. et. al., Texas Rep. Biol. Med. 9, 235 (1951), Bonhard, K., Arzneim.-Forsch. 21, 1667 (1971) sowie Nitschmann und Stoll, Pharm.-Ztg. 42, 1594 (1968) beschrieben.

Polygeline wird durch Vernetzen von Rindergelatine mit Hexamethylendiisocyanat gewonnen und wird von Schmidt-Thome, J., A. Mager und H. H. Schöne, Arzneim.-Forsch. 12, 378 (1962) beschrieben.

Succinoyl-Gelatine wird durch Kondensation von Gelatine und Bernsteinsäureanhydrid gewonnen und ist in US 2 827 419, GB 836 082 sowie von Tourtelotte, D. und H. E. Williams, in: Stainsby, G., Gelatin and Glue Research, 1958 beschrieben.

Bevorzugt bezieht sich die vorliegende Erfindung auf eine neue Form der Injektion von Muzolimin, bestehend aus einer Konzentrat-und einer Verdünnungslösung, die vor Applikation gemischt werden und dabei hinreichend stabile, übersättigte Lösungen bilden.

Die erfindungsgemäßen parenteralen Lösungen können Muzolimin beispielsweise in einer bis zu etwa 70fach höheren Konzentration enthalten als in entsprechenden wäßrigen Lösungen.

Weitere Bestandteile des Konzentrates sind ein oder mehrere zur intravenösen Injektion grundsätzlich geeignete organische Lösungsmittel wie Ethanol, 1,2-Propylenglykol, Polyethylenglykol mit Molekulargewichten zwischen 200 und 600, Glycerin oder Tetrahydrofurfurylalkoholpolyethylenglykolether in Konzentrationen von 30 bis 99,999 % G/V.

Das Konzentrat kann 0,1 bis 30 % G/V modifizierte Gelatine mit einem Molekulargewicht zwischen 10.000 und 50.000 wie Oxypolygelatine oder Polymerisat abgebauter succinoylierter Gelatine und/oder bis zu 70 % G/V Wasser für Injektionszwecke enthalten. Darüber hinaus können weitere, in parenteralen Lösungen übliche Hilfsstoffe zugesetzt werden.

Die Verdünnungslösung enthält 0,1 bis 30 % G/V modifizierte Gelatine mit einem Molekulargewicht zwischen 10.000 und 50.000 wie Oxypolygelatine oder Polymerisat abgebauter succinoylierter Gelatine, 10 bis 99,9 % G/V Wasser für Injektionszwecke sowie gegebenenfalls weitere, in parenteralen Lösungen übliche Hilfsstoffe.

Übliche Hilfsstoffe für parenterale Lösungen sind beispielsweise:
Säuren, Basen oder Puffersubstanzen zur pH-Einstellung, Salze, Zucker oder mehrwertige Alkohole zur Isotonisierung, Konservierungsmittel wie Benzylalkohol, Chlorbutanol, Antioxidantien wie Sulfite, Acetylcystein oder Ascorbinsäure.

Enthält das Konzentrat bereits modifizierte Gelatine, kann dies in der Verdünnungslösung entfallen.

Es wurde auch ein Verfahren zur Herstellung der erfindungsgemäßen parenteralen Lösungen von Muzolimin gefunden, das dadurch gekennzeichnet ist, daß man das Muzolimin in einem Lösungsmittel löst und dann Wasser, modifizierte Gelatine und übliche Hilfsstoffe zusetzt.

In einer bevorzugten Ausführungsform des erfindungsgemäßen Verfahrens stellt man eine Konzentrat-und eine Verdünnungslösung her, die vor Applikation gemischt werden und dabei eine stabile, übersättigte Lösung bilden.

Die bevorzugte Ausführungsform des erfindungsgemäßen Verfahrens ist dadurch gekennzeichnet, daß man zur Herstellung der Konzentratlösung das Arzneimittel unter Rühren in einem organischen Lösungsmittel löst und dann Wasser, modifizierte Gelatine und übliche Hilfsstoffe zusetzt und zur Herstellung der Verdünnungslösung modifizierte Gelatine in Wasser löst und dann die Konzentratlösung und die Verdünnungslösung mischt.

Organische Lösungsmittel zur Lösung von Muzolimin sind beispielsweise:
Ethanol, 1,2-Propylenglykol, Polyethylenglykol mit Molekulargewichten zwischen 200 und 600, Glycerin oder Tetrahydrofurfurylalkoholpolyethylenglykolether.

Überraschenderweise sind die erfindungsgemäßen parenteralen Lösungen schwer löslicher Arzneimittel stabil und können ohne Probleme angewendet werden.

Beispiel 1

0,6 kg Muzolimine werden unter Rühren und Stickstoffbegasung in 30,0 kg Ethanol 96 % und 30,0 kg Polyethylenglykol 400 gelöst. Nach Zusatz von 30,0 kg Wasser für Injektionszwecke werden 0,05 kg Natriummetabisulfit unter Rühren gelöst und der Ansatz mit Wasser für Injektionszwecke auf 100 Liter aufgefüllt. Die Lösung wird filtriert, unter Stickstoffbegasung in Ampullen zu 5 ml abgefüllt und hitzesterilisiert (Konzentrat).

5,5 kg Oxypolygelatine, 0,584 kg Natriumchlorid und 0,019 kg Ethylendiamintetraessigsäure (Dinatriumsalz) werden in 80 kg Wasser für Injektionszwecke gelöst und der Ansatz mit Wasser für Injektionszwecke auf 100 Liter aufgefüllt.

Die Lösung wird filtriert, unter Stickstoffbegasung in Ampullen zu 5 ml abgefüllt und hitzesterilisiert (Verdünnungslösung).

Das Injektionssystem besteht aus einer Konzentratampulle und einer Ampulle mit Verdünnungslösung und ermöglicht die intravenöse Applikation von 30 mg Muzolimine als klare, physiologisch verträgliche Lösung.

Beispiel 2

0,4 kg Muzolimine werden unter Rühren und Stickstoffbegasung in 30,0 kg Ethanol 96 % und 35,0 kg 1.2-Propylenglykol gelöst. Nach Auffüllen mit Wasser für Injektionszwecke auf 100 Liter wird filtriert, unter Stickstoffbegasung in Ampullen zu 5 ml abgefüllt und hitzesterilisiert (Konzentrat).

4 kg Gelatine-Polysuccinat mit einem mittleren Molekulargewicht von 30 000 und einem Succinylierungsgrad von 0,026 werden zusammen mit 0,3675 kg Natriumacetattrihydrat, 0,1607 kg Natriumchlorid, 0,0403 kg Kaliumchlorid, 0,0133 kg Calciumchloriddihydrat, 0,0204 kg Magnesiumchloridhexahydrat, 0,02 kg Bernsteinsäure, 0,302 kg Natriumhydroxid und 0,517 kg konzentrierter Salzsäure in 50 kg Wasser für Injektionszwecke gelöst und der Ansatz mit Wasser für Injektionszwecke auf 100 Liter aufgefüllt. Die Lösung wird filtriert, in Ampullen zu 5 ml abgefüllt und hitzesterilisiert (Verdünnungslösung).

Anwendungsbeispiele:

Beispiel 3

Zur Formulierung einer stabilen, bei Lagerung nicht auskristallisierenden Injektionslösung von 20 mg Muzolimine in 10 ml Volumen müssen 40 % G/V Ethanol oder 70 % G/V Polyethylenglykol 400 oder 70 % G/V 1.2-Propylenglykol oder 47 % G/V einer Mischung aus Ethanol (20 % G/V) und Polyethylenglykol 40 (27 % G/V) als Lösungsvermittler zugesetzt werden. Derartige Lösungsmittelkonzentrationen sind physiologisch unverträglich. So führt die Applikation von 10 ml Lösung, die 0,02 % G/V Muzolimine, 20 % G/V Ethanol und 27 % G/V Polyethylenglykol 400 enthält, am Hund zu erheblichen Unverträglichkeitsreaktionen wie Hämatome im Injektionsbereich, Schwellung und Verhärtung der Venen.

Stellt man ein Konzentrat her, welches in 5 ml Volumen 0,4 % G/V Muzolimine, 30 % G/V Ethanol und 30 % G/V Polyethylenglykol 400 enthält, so tritt beim Verdünnen mit Wasser im Verhältnis 1:1 auf physiologisch akzeptable Lösungsmittelkonzentrationen Trübung der Lösung ein. Eine derartige Lösung darf nicht intravenös injiziert werden.

Wird dasselbe Konzentrat mit 5 ml Verdünnungslösung verdünnt, die 5,5 % G/V Oxypolygelatine enthält, entsteht eine selbst bei +5°C über mehrere Stunden klare und physiologisch verträgliche Injektionslösung. So konnten auch nach sieben Injektionen an aufeinanderfolgenden Tagen von 10 ml dieser Lösung am Hund keinerlei Unverträglichkeitsreaktionen festgestellt werden.

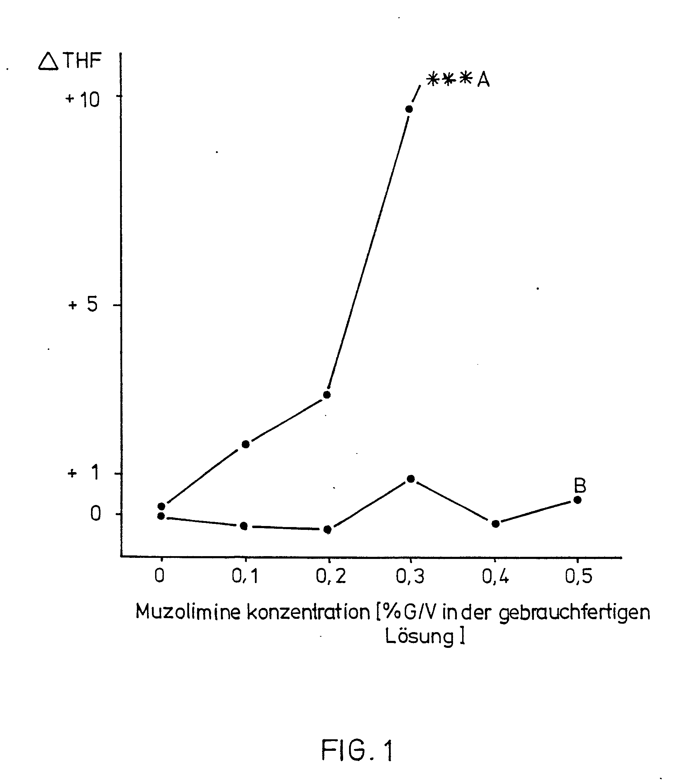

Fig. 1 demonstriert die Wirksamkeit des erfindungsgemäßen Injektionssystems: Während bei Verdünnung des Konzentrats mit Wasser schon bei Muzoliminekonzentrationen von 0,1 % G/V Trübung eintritt, sind Lösungen des Injektionssystems auch bei Muzolimine-Konzentrationen von 0,5 % G/V klar. Die mit dem Injektionssystem erreichte gelöste Arzneistoffkonzentration beträgt etwa das 70-fache der Löslichkeit von Muzolimine in Wasser (Raumtemperatur) und das 11-fache der Löslichkeit von Muzolimine im selben Lösungsmittelgemisch (+5°C).

In Fig. 1 haben die Symbole die folgende Bedeutung:

A = Verdünnung des Konzentrates mit Wasser

B = Verdünnung des Konzentrates mit Oxypolygelatinelösung

‴ = Ausfallen grober Wirkstoffkristalle

ΔTHF = Veränderung der Trübung in Trübungseinheiten/Formazin nach DIN 38 404 C2.

## Ansprüche

1. Parenterale Lösungen von 5-Amino-2-[1-(3,4-dichlorophenyl)-ethyl]-2,4-di-hydro-3H-pyrazol-3-on, enthaltend modifizierte Gelatine.

2. Parenterale Lösungen nach den Anspruch 1, bestehend aus einer 5-Amino-2-[1-(3,4-dichlorophenyl)-ethyl]-2,4-di-hydro-3H-pyrazol-3-on enthaltenden Konzentratlösung und einer Verdünnungslösung.

3. Parenterale Lösungen nach den Ansprüchen 1 und 2, dadurch gekennzeichnet, daß die Konzentratlösung 0,1 bis 30 % G/V modifizierte Gelatine enthält.

4. Parenterale Lösungen nach den Ansprüchen 1 bis 3, dadurch gekennzeichnet, daß die Verdünnungslösung 0,1 bis 30 % G/V modifizierte Gelatine enthält.

5. Parenterale Lösungen nach den Ansprüchen 1 bis 4, dadurch gekennzeichnet, daß modifizierte Gelatine mit einem mittleren Molekulargewicht im Bereich von 10.000 bis 50.000 verwendet wird.

6. Verfahren zur Herstellung von parenteralen Lösungen von 5-Amino-2-[1-(3,4-dichlorophenyl)-ethyl]-2,4-di-hydro-3H-pyrazol-3-on dadurch gekennzeichnet, daß man das 5-Amino-2-[1-(3,4-dichlorophenyl)-ethyl]-2,4-di-hydro-3H-pyrazol-3-on in einem Lösungsmittel löst und dann Wasser, modifizierte Gelatine und übliche Hilfsstoffe zusetzt.

7. Verfahren zur Herstellung von parenteralen Lösungen nach Anspruch 6, dadurch gekennzeichnet, daß man zur Herstellung der Konzentratlösung das 5-Amino-2-[1-(3,4-dichlorophenyl)-ethyl]-2,4-di-hydro-3H-pyrazol-3-on unter Rühren in einem organischen Lösungsmittel löst und dann Wasser, modifizierte Gelatine und übliche Hilfsstoffe zusetzt und zur Herstellung der Verdünnungslösung modifizierte Gelatine in Wasser löst und dann die Konzentratlösung und die Verdünnungslösung mischt.

8. Verfahren nach den Ansprüchen 6 und 7, dadurch gekennzeichnet, daß man 1 Volumenteil der Konzentratlösung mit 0,2 bis 1000 Volumenteile der Verdünnungslösung mischt.

FIG. 1

| | |
|---|---|
| Europäisches Patentamt | **EUROPÄISCHER RECHERCHENBERICHT** |

Nummer der Anmeldung

EP 87 11 0842

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-2 319 278 (BAYER AG)<br>* Seite 11, Zeile 1 - Seite 13, Zeile 20; Seite 24, Beispiel 17 *<br>--- | 1-8 | A 61 K 9/08<br>A 61 K 47/00<br>A 61 K 31/415 |
| Y | EP-A-0 187 361 (HOECHST AG)<br>* Seite 3, Zeile 29 - Seite 7, Zeile 37 *<br>--- | 1-8 | |
| A | EP-A-0 143 305 (BAYER AG)<br>* Ansprüche 1-6 *<br>--- | | |
| A | EP-A-0 162 332 (SUNTORY LTD)<br>* Ansprüche 1-8 *<br>--- | | |
| A | HAGERS HANDBUCH DER PHARMAZEUTISCHEN PRAXIS, Band 7, Teil A: "Arzneiformen und Hilfsstoffe", 4. Auflage, 1971, Seiten 411-412, Springer Verlag, Berlin, DE<br>* Seite 411, Zeile 22 - Seite 412, Zeile 36 *<br>--- | | |
| A | DE-A-2 519 723 (J.G. GOW et al.)<br>* Anspruch 1 *<br>--- | | RECHERCHIERTE SACHGEBIETE (Int. Cl.4)<br><br>A 61 K |
| A | FR-A-2 189 057 (SAVAGE LABORATORIES INC.)<br>* Anspruch 1 *<br>----- | | |

Der vorliegende Recherchenbericht wurde für alle Patentansprüche erstellt

| Recherchenort | Abschlußdatum der Recherche | Prüfer |
|---|---|---|
| DEN HAAG | 19-11-1987 | TZSCHOPPE,D.A. |